# EUROPÄISCHE PATENTSCHRIFT

(11) **EP 1 296 669 B1**
(45) Veröffentlichungstag und Bekanntmachung des Hinweises auf die Patenterteilung: **14.12.2005**
(21) Anmeldenummer: 01929617.7
(22) Anmeldetag: 03.05.2001
(51) Int. Cl.: A61K 31/202, A61K 31/201, A61K 35/60, A61P 17/06, A61P 11/12

(54) **KOMBINATIONSPRÄPARAT AUS omega-3-FETTSÄUREN UND KONJUGIERTEN LINOLSÄUREN ZUR BEHANDLUNG IMMUNOLOGISCH GEPRÄGTER KRANKHEITSBILDER**
COMBINATION PREPARATION CONSISTING OF OMEGA-3-FATTY ACIDS AND OF CONJUGATED LINOLEIC ACIDS FOR TREATING IMMUNOLOGICALLY ORIENTED CLINICAL SIGNS
PREPARATION COMBINANT DES ACIDES GRAS OMEGA-3 ET DES ACIDES LINOLENIQUES CONJUGUES POUR LE TRAITEMENT DE MALADIES CARACTERISEES PAR DES TABLEAUX CLINIQUES A COMPOSANTE IMMUNOLOGIQUE MARQUEE

(30) Priorität: 05.05.2000 DE 10022001
(43) Veröffentlichungstag der Anmeldung: 02.04.2003
(73) Patentinhaber: Fresenius Kabi Deutschland GmbH, 61352 Bad Homburg v.d.H. (DE)
(72) Erfinder: SOMMERMEYER, Klaus, 61191 Rossbach (DE)
(74) Vertreter: Luderschmidt, Schüler & Partner GbR
(86) Internationale Anmeldenummer: PCT/EP2001/005011
(87) Internationale Veröffentlichungsnummer: WO 2001/085161

(56) Entgegenhaltungen:
- WO-A-93/21912
- WO-A-98/17269
- WO-A-99/08540
- DE-A- 4 432 633

## Beschreibung

Die vorliegende Erfindung betrifft die kombinierte Verwendung von einerseits ω-3-Fettsäuren und/oder deren pharmazeutisch verträglichen Salzen und/oder Estern und/oder einem an ω-3-Fettsäuren im Triglyceridverband angereicherten Fischöl und andereseits von konjugierten Linolsäuren (CLA) und/oder deren pharmazeutisch verträglichen Salzen und/oder Estern zur Herstellung von pharmazeutischen Präparaten, mit welchen nach oraler und/oder parenteraler Verabreichung eine Verstärkung der immunmodulierenden und antiinflammatorischen Wirkungen erreicht werden kann, die man bei Krankheitsbildern mit ausgeprägter immunologischer Komponente erzielt, wenn man die ω-3-Fettsäuren und/oder deren Derivate ohne CLA und/oder deren Derivate einsetzt.

Mehrfach ungesättigte Fettsäuren sind seit langem als Zusatz zu oral und/oder parenteral verabreichter klinischer Ernährung zur Beeinflussung des Krankheitsverlaufs bei kritisch Kranken bekannt. Die Zufuhr ungesättigter Fettsäuren über die Nahrung ist für den Körper sowohl von struktureller als auch funktioneller Bedeutung. Ihre strukturelle Bedeutung liegt in der Beeinflussung des Fettsäuremusters der Zellmembranen und ihre funktionelle Bedeutung in der Bereitstellung bestimmter Mediatoren, d.h von hormonähnlichen Wirkstoffen, welche physiologische Prozesse modulieren. Insbesondere die Wirkungen auf das Immunsystem, wie z.B. bei entzündlichen und allergischen Reaktionen, und auf das vaskuläre System machen mehrfach ungesättigte Fettsäuren für eine Ernährung mit therapeutischen Zielen interessant.

Die Druckschrift WO99/08540 beschreibt die Herstellung eines Produkts, welches konjugierte Linolensäuren und omega-3 Fettsäuren aufweist. Das Produkt kann zur Behandlung von Krankheiten eingesetzt werden.

Die Dokumente WO93/21912 und DE 44 32 633 beschreiben pharmazeutische Präparate, die omega-3 Fettsäuren enthalten.

Die Druckschrift W098/98/17269 beschreibt die Verwendung von Zinksalzen von konjugierten Linolensäuren zur Behandlung von Hautkrankheiten.

Die ungesättigten Fettsäuren werden je nach der Position ihrer ersten Doppelbindung nach dem 9., 6. oder 3. Kohlenstoffatom (vom Methylende her gezählt) in ω-9-, ω-6- und ω-3-Fettsäuren unterschieden. Sie leiten sich von den entsprechenden C₁₈-Fettsäuren ab, und zwar ω-9-Fettsäuren von der Ölsäure (*cis*-9-Octadecensäure), ω-6-Fettsäuren von der Linolsäure (*cis,cis*-9-12-Octadecadiensäure) und ω-3-Fettsäuren von der α-Linolensäure (*cis,cis,cis*-9-12-15-Octadecatriensäure). Während die ω-9-Fettsäuren vom Menschen selbst synthetisiert werden können, sind ω-6- und ω-3-Fettsäuren essentiell und müssen mit der Nahrung zugeführt werden. Dies beruht darauf, daß Säugetiere prinzipiel nicht mehr in der Lage sind, jenseits der Δ⁹-Position (Zählung vom carboxylseitigen Ende her) weitere Doppelbindungen in Fettsäuren einzuführen.

In der Nahrung liegen die ω-6-Fettsäuren hauptsächlich als Linolsäure (C₁₈-Säure) oder als Arachidonsäure (all-*cis*-5,8,11,14-Eicosatetraensäure) vor und die ω-3-Fettsäuren hauptsächlich in Form von α-Linolensäure (aus Pflanzenölen und tierischen Fetten) sowie von Eicosapentaensäure (5,8,11,14,17-Eicosapentaensäure) und Docosahexaensäure (aus Fischölen).

Von diesen polyungesättigten Fettsäuren haben die C₂₀-Säuren Arachidonsäure (AA) und Eicosapentaensäure (EPA) eine besondere Bedeutung für die Wirkung auf den Immunstatus, weil sie die Vorstufen zur Bildung der immunmodulatorisch wirkenden Eicosanoide darstellen, worunter die Leukotriene und Prostanoide ( Prostaglandine, Prostacycline, Thromboxane) zu subsummieren sind.

Sowohl AA als auch EPA werden überwiegend in Phospholipide der Zellmembran eingebaut, aus denen sie je nach Bedarf durch Phospholipase A₂ freigesetzt werden können. In Abhängigkeit vom spezifischen Enzymmuster einer jeweiligen Zelle werden sie dann in verschiedene Eicosanoide umgewandelt. Dabei können zwei verschiedene Stoffwechselwege beschritten werden, entweder der Cyclooxygenaseweg oder der Lipoxygenaseweg, wo beide Fettsäuren jeweils um das Enzym Cyclooxygenase bzw Lipoxygenase miteinander konkurrieren und sich gegenseitig verdrängen können. Über den Cyclooxygenaseweg werden die Prostanoide und entsprechend über den Lipoxygenaseweg die Leukotriene gebildet.

Dabei werden aus AA unter der Einwirkung von Lipoxygenase Leukotriene der 4er Serie (z.B. LTB₄, LTC₄, LTD₄) und aus EPA Leukotriene der 5er Serie (z.B. LTB₅, LTC₅, LTD₅) gebildet, die sich in ihren Wirkungen unterscheiden. Entsprechend werden unter Einwirkung der Cyclooxygenase aus AA Prostanoide der 2er Serie (z.B. TXA₂, PGE_{2.} PGI₂) und aus EPA Prostanoide der 3er Serie (z.B. TXA₃, PGE₃, PGI₃) gebildet, die sich ebenfalls in ihrer Wirkung unterscheiden. In Abhängigkeit vom Verhältnis von ω-3- zu ω-6-Fettsäuren und vom Stoffwechselweg ergeben sich dann in den Geweben bzw. Zellen (z.B. in Lungen, Leber, Nieren) unterschiedliche Wirkungsmuster von Eicosanoiden, was bei der Therapie schwer kranker Patienten, z.B. bei entzündlichen Prozessen, ausgenutzt werden kann.

Die Eicosanoide, die in allen Zellen von Säugern (außer in Erythrozyten) als physiologische Reaktion auf einen Stimulus gebildet werden können, wirken schon in äußerst geringen Konzentrationen. Wegen ihrer äußerst kurzen Lebensdauer können sie ihre Wirkung jedoch nur in der Zelle entfalten, wo sie erzeugt wurden (autokrin) oder allenfalls noch in Nachbarzellen (parakrin), so daß sie "lokale Mediatoren" darstellen. Ihre Wirkung am Ort des Geschehens (einem Gewebe oder Organ) erfolgt über chemotaktische Vorgänge, wodurch eine Migration von Effektorzellen, z.B. Makrophagen oder neutrophilen Granulozyten, zum Entzündungsherd in Gang gesetzt wird. Bei schweren Krankheitsbildern, wie z.B. bei einer Sepsis, einer Psoriasis oder einer zystischen Fibrose kann es zu lokaler oder systemischer inflammatorischer Aktivierung des Stoffwechsels bis hin zu einer Hyperinflammation kommen. Als Folge davon können schwere Gewebsschäden bis hin zu einem Multiorganversagen auftreten.

Es konnte nun gezeigt werden, daß solche hyperinflammatorischen Prozesse, insbesondere bei Psoriasis und zystischer Fibrose, durch erhöhte Verabreichung von ω-3-Fettsäuren gegenüber ω-6-Fettsäuren abgeschwächt werden konnten. Der Grund ist darin zu sehen, daß die aus ω-3-Fettsäuren bzw. EPA gebildeten Eicosanoide eine geringere proinflammatorische Wirkung ausüben als solche aus ω-6-Fettsäuren bzw. AA gebildete Eicosanoide. Aus der vermehrten Nutzung von EPA zur Bildung der entsprechenden Eicosanoide auf Kosten einer entsprechenden Nutzung der AA zur Eicosanoidbildung resultiert dann ein antiinflammatorischer Nettoeffekt. Durch die Bereitstellung zusätzlicher ω-3-Fettsäuren wird das Verhältnis von ω-3- zu ω-6-Fettsäuren zugunsten der ersteren verschoben, so daß mehr Eicosanoide aus ω-3-Fettsäuren gebildet werden. Während also z.B. durch die Bildung von Thromboxan A₂ und Leukotrien B₄ aus ω-6-Fettsäuren bzw. AA die Entzündungsreaktion verstärkt wird, läßt sie sich durch eine vermehrte Bildung von Thromboxan A₃ und Leukotrien B₅ aus ω-3-Fettsäuren bzw. EPA (auf Kosten von Thromboxan A₂ und Leukotrien B₄) wegen der geringeren inflammatorischen Wirkung der EPA-Derivate bremsen.

In größeren Mengen üben Eicosanoide eine supprimierende Wirkung auf die zelluläre Immunantwort aus. So bremsen aus AA (bzw. ω-6-Fettsäuren) gebildetes Prostaglandin E₂ und Leukotrien B₄ in hoher Konzentration die zelluläre Abwehrreaktion, weil von diesen Eicosanoiden die Produktion von für die Proliferation der T- und B-Zellen essentiellem Interleukin 2 gehemmt wird. Auch hier weisen die entsprechenden aus EPA gebildeten Eicosnanoide eine im Vergleich mit den aus AA gebildeten Eicosanoiden geringere Hemmwirkung auf, so daß bei einem höheren Anteil von Eicosanoiden aus ω-3-Fettsäuren wieder eine positive immunmodulatorische Nettoreaktion resultiert.

Insgesamt wären also bei oraler oder parenteraler Verabreichung von mehrfach ungesättigten Fettsäuren umso bessere immunmodulierende und antiinflammatorische Effekte bei kritischen Krankheitsbildern zu erwarten, je mehr aus ω-3-Fettsäuren bzw. EPA stammende Eicosanoide im Verhältnis zu solchen aus ω-6-Fettsäuren bzw. AA stammenden Eicosanoiden gebildet werden. Es zeigte sich jedoch, daß sich über eine Einstellung des ω-3/ω-6-Verhältnisses mit der zugeführten Nahrung nur ein maximal erreichbares LTC₅/LTC₄-Verhältnis - und damit der vorteilhafteste immunmodulierende Effekt - erreichen ließ, wenn eine Fettemulsion mit einem ω-3/ω-6-Verhältnis von 1:2 eingesetzt wurde. Eine weitere Erhöhung des ω-3-Fettsäureanteils in der verabreichten Fettemulsion erbrachte keine Verbesserung im LTC₅/LTC₄-Verhältnis. Der Grund ist darin zu suchen, daß die in stimulierten Zellen für die Bereitstellung der Eicosanoide "verbrauchte" Arachidonsäure ständig aus Linolsäure nachsynthetisiert wird. Linolsäure ist eine zweifach ungesättigte C₁₈-Fettsäure (*cis,cis-*9,12-Octadecadiensäure) welche in Form ihrer Glycerinester Bestandteil praktisch aller fetten Öle ist. Linolsäure ist z.B. in Sonnenblumenöl zu 61% und in Sojaöl zu 53% enthalten. Linolsäure ist ferner für den Aufbau der Zellmembran unerläßlich und stellt für den Menschen eine essentielle Fettsäure dar.

Die derzeit in der Therapie eingesetzten Präparate mit entsprechenden immunmodulatorischen und antiinflammatorischen Wirkungen weisen, da sie nicht als lokale Mediatoren anzusehen sind, mehr oder weniger große Nebenwirkungen auf. Da aber die mit der Verabreichung von reinen ω-3-Fettsäuren (ohne ω-6-Fettsäuren) zu erzielenden immunmodulatorischen und antiinflammatorischen Wirkungen nicht an die mit den etablierten Arzeimitteln derzeit erzielbaren Wirkungen heranreichen, konnten entsprechende Präparate auf ω-3-Fettsäurebasis nur unterstützend zu den etablierten Präparaten eingesetzt werden.

Andererseits wären aber die wegen der lokal gebildeten und daher auch nur lokal wirkenden Mediatoren zu erwartenden äußerst geringen Nebenwirkungen dann ein entscheidender Fortschritt, wenn man Präparate zur Verfügung hätte, die auf der Grundlage von mehrfach ungesättigten Fettsäuren auch als Zusatz zu einem oral oder parenteral verabreichbaren Nahrungsmittel eine den etablierten Präparaten vergleichbare pharmazeutische Wirkung zeigen würden.

Der Erfindung lag daher die Aufgabe zugrunde, solche den immunmodulatorischen und antiinflammatorischen Wirkungen von etablierten Präparaten in ihrer Wirkung vergleichbare Präparate auf Basis von ungesättigten Fettsäuren zur Verfügung zu stellen, welche nach wie vor die auf der Bildung von lokalen Mediatoren beruhenden Vorteile im Hinblick auf evtl. Nebenwirkungen aufweisen.

Diese Aufgabe wird durch Arzneimittel mit allen Merkmalen des Anspruchs 1 gelöst.

Eine Stoffwechselzwischenreaktion bei der obigen Biosynthese von Arachidonsäure aus Linolsäure besteht in einer Desaturierungsreaktion unter Mitwirkung des Enzyms Δ⁶-Desaturase, welches auch die analoge Desaturierungsreaktion bei der Biosynthese von Eicosatetraensäure aus α-Linolensäure katalysiert. Es wurde nun überraschend gefunden, daß eine Kombination von ω-3-Fettsäuren und konjugierten Linolsäuren (CLA) die im Hinblick auf die erwünschten immunmodulatorischen und antiinflammatorischen Wirkungen "ungünstigen", von der ω-6-Fettsäure Arachidonsäure herstammenden Eicosanoide (Prostanoide der 2er Serie und Leukotriene der 4er Serie) in ihrer Bildung zu Gunsten der antiinflammatorisch wirkenden, aus ω-3-Fettsäuren stammenden Mediatoren (Prostanoide der 3er Serie und Leukotriene der 5er Serie) zurückdrängen konnte.

Dadurch wird eine Wirkungsverstärkung der ω-3-Fettsäuren erreicht, was dem Kombinationspräparat eine ausreichende Verstärkung der immunmodulatorischen und antiinflammatorischen Wirkungen verleiht, die denen der etablierten Pharmaka nahekommen, jedoch ohne deren Nachteile hinsichtlich der Nebenwirkungen.

Die konjugierten Linolsäuren (CLA) lassen sich durch Isomerisierung aus Linolsäure nach an sich bekannten Verfahren herstellen. Bei der Isomerisierung werden zunächst die Doppelbindungen von Position 9 in die Position 10 oder von der Position 12 in die Position 11 unter Umkehrung der *cis, cis-* in die *cis, trans-* bzw. *trans, cis*-Formation verschoben. Bei dieser Verschiebung sind theoretisch 8 verschiedene geometrische Isomere möglich. Die Doppelbindungen können sich bei den Isomerisierungsvorgängen in weitere Positionen verschieben. Dabei können gleichzeitig *cis, trans*-Formationsumwandlungen auftreten, so daß eine größere Anzahl von Positionsisomeren entstehen. Eines dieser Isomere bewirkt die spezifische Hemmung der Δ⁶-Desaturierung von Linolsäure. Die CLA werden erfindungsgemäß in Form ihrer freien Säuren, vorzugsweise jedoch in Form ihrer pharmazeutisch verträglichen Ester, insbesondere als Triglyceride, eingesetzt.

ω-3-Fettsäuren kommen bekanntlich in Fischölen im Triglyceridverband vor. Fischöle sind besonders reich an EPA und DHA, sie können aber auch Docosapentaensäure (DPA) enthalten. Diese ω-3-Fettsäuren bzw. die entsprechenden Triglyceride lassen sich aus Fischölen in an sich bekannter Weise isolieren.

Die ω-3-Fettsäuren werden erfindungsgemäß in Form ihrer freien Säure oder ihrer pharmazeutisch verträglichen Ester, bevorzugt jedoch im Triglyceridverband, eingesetzt, wobei die erfindungsgemäß verwendeten ω-3-Fettsäuren 15-50 Gew.-% EPA, 10-50 Gew.-% DHA und 2-8 Gew.-% DPA im Triglyceridverband enthalten.

Soll das Kombinationspräparat parenteral verabreicht werden, ist es besonders vorteilhaft, die ω-3-Fettsäuren als im Triglyceridverband angereicherte Fischöle einzusetzen. Die Herstellung deratig hochraffinierter Fischöle wird z.B. in der DE 37 22 540 beschrieben.

Die Herstellung eines therapeutisch wirksamen Kombinationspräparats erfolgt so, daß die beiden wirksamen Komponenten, nämlich einerseits ω-3-Fettsäuren oder deren pharmazeutisch verträgliche Ester oder ein an ω-3-Fettsäuren im Triglyceridverband angereichertes Fischöl und andererseits die konjugierten Linolsäuren (CLA) oder deren pharmazeutisch verträgliche Ester bzw. Triglyceride in vorbestimmtem Mengenverhältnis zu einer Fettemulsion verarbeitet werden, die dann entweder oral oder parenteral dem Patienten zugeführt wird.

In dem Kombinationspräparat soll das Gewichtsverhältnis von ω-3-Fettsäuren und/oder deren pharmazeutisch verträglichen Salzen und/oder Estern zu den konjugierten Linolsäuren (CLA) und/oder deren pharmazeutisch verträglichen Salzen und/oder Estern vorzugsweise im Bereich von 1:5 bis 10:1 liegen, am meisten bevorzugt bei 1:1. Werden die ω-3-Fettsäuren in Form von im Triglyceridverband angereichertem Fischöl eingesetzt und die CLA in Form von pharmazeutisch verträglichen Estern, wie Triglyceriden, liegt das gewichtsprozentuale Verhältnis von angereichertem Fischöl zu CLA oder ihren Estern vorzugsweise im Bereich von 1:5 bis 20:1.

In einer bevorzugten Ausführungsform werden 0,3-40 Gew.-% ω-3-Fettsäuren in Form ihrer Derivate, vorzugsweise in Form der ω-3-Fettsäuretriglyceride, oder 0,5-40 Gew.-% eines 25-80% ω-3-Fettsäuren im Triglyceridverband enthaltenden Fischöls und 0,5-20 Gew.-% CLA und/oder deren Derivate, vorzugsweise in Form ihrer Triglyceride, als Komponenten eingesetzt.

Vorzugsweise werden die Komponenten gleichzeitig oder nacheinander vorbestimmten Substraten zugesetzt und in diesen homogen verteilt. Vorzugsweise werden als Substrate feste (bei oraler Applikation) oder flüssige Lebensmittel eingesetzt. Desweiteren werden vorzugsweise solche Lebensmittel eingesetzt, die selbst einen gewissen Fettanteil aufweisen, der vorzugsweise bei 5-30% liegen soll. Andere bevorzugte Substrate sind W/O-Emulsionen und O/W-Emulsionen, insbesondere solche, die für Infusionen geeignet sind, sowie bei oraler Applikation auch Weichgelatinekapseln, in welche die beiden wirksamen Komponenten des Präparats gemeinsam oder getrennt voneinander eingeschlossen werden.

Eine O/W-Infusionsemulsion für parenterale Verabreichung enthält zusätzlich noch für Injektionszwecke geeignetes destilliertes Wasser, 1-2 Gew.-% Eilecithin und 2-3 Gew.-% Glycerin, wobei 1-30 Gew.-%, vorzugsweise 1,5-20 Gew.-%, der Emulsion durch die erfindungsgemäße Kombination aus ω-3-Fettsäuren und CLA-Triglyceriden besteht. Wird als Neutralöl ein Fischöl eingesetzt, braucht wegen des Gehalts der darin enthaltenen ω-3-Fettsäuretriglyceride nur noch CLA-Triglyceride in der zur Erreichung des gewünschten Gewichtsverhältnisses von ω-3-Fettsäuren zu CLA erforderlichen Menge zugesetzt werden.

## Patentansprüche

1. Verwendung von ω-3-Fettsäuren und/oder deren pharmazeutisch verträglichen Salzen und/oder Estern und/oder einem an ω-3-Fettsäuren im Triglyceridverband angereicherten Fischöl zusammen mit konjugierten Linolsäuren (CLA) und/oder deren pharmazeutisch verträglichen Salzen und/oder Estern, wobei die eingesetzten ω-3-Fettsäuren sich aus 15-50 Gew.-% Eicosapentaensäure, 10-50 Gew.-% Docosahexaensäure und 2-8 Gew.-% Docosapentaensäure zusammensetzen, zur Herstellung eines Arzneimittels mit immunmodulatorischer und antiinflammatorischer Wirkung gegen Krankheiten mit immunologischer Ausprägung.

2. Verwendung nach Anspruch 1, wobei ω-3-Fettsäuretriglyceride als ω-3-Fettsäureester eingesetzt werden.

3. Verwendung nach Anspruch 1, wobei an ω-3-Fettsäuren im Triglyceridverband angereichertes Fischöl eingesetzt wird.

4. Verwendung nach Anspruch 1 oder 2, wobei das Gewichtsverhältnis von ω-3-Fettsäuren und/oder deren pharmazeutisch verträglichen Salzen und/oder Estern zu den konjugierten Linolsäuren (CLA) und/oder deren pharmazeutisch verträglichen Salzen und/oder Estern im Bereich von 1:5 bis 10:1 liegt.

5. Verwendung nach Anspruch 4, wobei das Gewichtsverhältnis von ω-3-Fettsäuren und/oder deren pharmazeutisch verträglichen Salzen und/oder Estern zu den konjugierten Linolsäuren (CLA) und/oder deren pharmazeutisch verträglichen Salzen und/oder Estern 1:1 ist.

6. Verwendung nach Anspruch 3, wobei das Gewichtsverhältnis von an ω-3-Fettsäuren im Triglyceridverband angereichertem Fischöl zu den konjugierten Linolsäuren (CLA) in Form von deren pharmazeutisch verträglichen Estern im Bereich von 1:5 bis 20:1 liegt.

7. Verwendung nach Anspruch 1, wobei 0,3-40 Gew.-% der ω-3-Fettsäuren in Form ihrer Triglyceride und 0,5-20 Gew.-% CLA in Form ihrer pharmazeutisch verträglichen Ester eingesetzt werden.

8. Verwendung nach Anspruch 1, wobei 0,5-40 Gew.-% eines 25-80% ω-3-Fettsäuren im Triglyceridverband enthaltenden Fischöls und 0,5-20 Gew.-% CLA in Form ihrer Triglyceride eingesetzt werden.

9. Verwendung nach einem der Ansprüche 1 bis 8 zum Einsatz gegen Psoriasis.

10. Verwendung nach einem der Ansprüche 1 bis 8 zum Einsatz gegen zystische Fibrose.

## Claims

1. Use of ω-3-fatty acids and/or their pharmaceutically compatible salts and/or esters and/or a fish oil enriched in ω-3-fatty acids in the triglyceride bond together with conjugated linoleic acids (CLA) and/or their pharmaceutically compatible salts and/or esters, whereby the employed ω-3-fatty acids consist of 15-50 wt.% eicosapentaenic acid, 10-50 wt.% docosahexaenic acid and 2-8 wt.% docosapentaenic acid, for the preparation of a drug with an immune-modulatory and anti-inflammatory action against diseases with an immunological character.

2. The use according to claim 1, whereby ω-3-fatty acid triglycerides are used as ω-3-fatty acids.

3. The use according to claim 1, whereby fish oil enriched in ω-3-fatty acids in the triglyceride bond is used.

4. The use according to claim 1 or 2, whereby the weight ratio of ω-3-fatty acids and/or their pharmaceutically compatible salts and/or esters to the conjugated linoleic acids (CLA) and/or their pharmaceutically compatible salts and/or esters lies in the range from 1:5 to 10:1.

5. The use according to claim 4, whereby the weight ratio of ω-3-fatty acids and/or their pharmaceutically compatible salts and/or esters to the conjugated linoleic acids (CLA) and/or their pharmaceutically compatible salts and/or esters is 1:1.

6. The use according to claim 3, whereby the weight ratio of fish oil enriched in ω-3-fatty acids in the triglyceride bond to the conjugated linoleic acids (CLA) in the form of their pharmaceutically compatible esters lies in the range from 1:5 to 20:1.

7. The use according to claim 1, whereby 0.3-40 wt.% of the ω-3-fatty acids in the form of their triglycerides and 0.5-20 wt.% CLA in the form of their pharmaceutically compatible esters are used.

8. The use according to claim 1, whereby 0.5-40 wt.% of a fish oil containing 25-80% ω-3-fatty acids in the triglyceride bond and 0.5-20 wt.% CLA in the form of their triglycerides are used.

9. The use according to any one of claims 1 to 8 for use against psoriasis.

10. The use according to any one of claims 1 to 8 for use against cystic fibrosis.

## Revendications

1. Utilisation d'acides gras oméga-3 et/ou de leurs sels et/ou esters pharmaceutiquement acceptables et/ou d'une huile de poisson enrichie en acides gras oméga-3 dans le complexe triglycéridique ensemble avec des acides linoléiques conjugués (CLA) et/ou leurs sels et/ou esters pharmaceutiquement acceptables, les acides gras oméga-3 utilisés se composant de 15 à 50% en poids d'acide eicosapentaénoique, de 10 à 50% en poids d'acide docosahexaénoïque et de 2 à 8% en poids d'acide docosapentaénoïque, pour préparer un médicament ayant une action immunomodulatrice et anti-inflammatoire contre des maladies de nature immunologique.

2. Utilisation selon la revendication 1, dans laquelle les triglycérides d'acides gras oméga-3 sont utilisés sous la forme d'esters d'acides gras oméga-3.

3. Utilisation selon la revendication 1, dans laquelle on utilise de l'huile de poisson enrichie en acides gras oméga-3 dans le complexe triglycéridique.

4. Utilisation selon la revendication 1 ou la revendication 2, dans laquelle le rapport en poids des acides gras oméga-3 et/ou de leurs sels et/ou esters pharmaceutiquement acceptables sur les acides linoléiques conjugués (CLA) et/ou leurs sels et/ou esters pharmaceutiquement acceptables se situe dans la plage de 1:5 à 10:1.

5. Utilisation selon la revendication 4, dans laquelle le rapport en poids des acides gras oméga-3 et/ou de leurs sels et/ou esters pharmaceutiquement acceptables sur les acides linoléiques conjugués (CLA) et/ou leurs sels et/ou esters pharmaceutiquement acceptables est de 1:1.

6. Utilisation selon la revendication 3, dans laquelle le rapport en poids de l'huile de poisson enrichie en acides gras oméga-3 dans le complexe triglycéridique sur les acides linoléiques conjugués (CLA) sous la forme de leurs esters pharmaceutiquement acceptables se situe dans la plage de 1:5 à 20:1.

7. Utilisation selon la revendication 1, dans laquelle on utilise de 0,3 à 40% en poids des acides gras oméga-3 sous la forme de leurs triglycérides et de 0,5 à 20% en poids de CLA sous la forme de leurs esters pharmaceutiquement acceptables.

8. Utilisation selon la revendication 1, dans laquelle on utilise de 0,5 à 40% en poids d'une huile de poisson contenant de 25 à 80% d'acides gras oméga-3 dans le complexe triglycéridique et de 0,5 à 20% en poids de CLA sous la forme de leurs triglycérides.

9. Utilisation selon l'une des revendications 1 à 8, destinée à être mise en oeuvre contre le psoriasis.

10. Utilisation selon l'une des revendications 1 à 8, destinée à être mise en oeuvre contre la fibrose cystique.
